(19) Europäisches Patentamt
European Patent Office
Office européen des brevets

(11) Veröffentlichungsnummer: **0 257 503**
**B1**

(12) # EUROPÄISCHE PATENTSCHRIFT

(45) Veröffentlichungstag der Patentschrift: **23.05.90**

(51) Int. Cl.[5]: **C07C 41/02**, C07C 43/23, C07C 39/11, C07D 311/72

(21) Anmeldenummer: **87111876.6**

(22) Anmeldetag: **17.08.87**

(54) **Verfahren zur Herstellung von optisch aktiven Hydrochinonderivaten, sowie von d-alpha-Tocopherol.**

(30) Priorität: **25.08.86 CH 3408/86**

(43) Veröffentlichungstag der Anmeldung:
**02.03.88 Patentblatt 88/9**

(45) Bekanntmachung des Hinweises auf die Patenterteilung:
**23.05.90 Patentblatt 90/21**

(84) Benannte Vertragsstaaten:
**AT BE CH DE FR GB IT LI NL**

(56) Entgegenhaltungen:
**EP-A- 0 065 368**
**EP-A- 0 129 252**
**EP-A- 0 173 142**
**US-A- 4 189 612**

(73) Patentinhaber: **F. HOFFMANN-LA ROCHE AG, Postfach 3255, CH-4002 Basel(CH)**

(72) Erfinder: **Barner, Richard, Dr., Traubenweg 16, CH-4108 Witterswil(CH)**
Erfinder: **Hübscher, Josef, Spausel 1080, CH-5703 Seon(CH)**

(74) Vertreter: **Urech, Peter, Dr. et al, Grenzacherstrasse 124 Postfach 3255, CH-4002 Basel(CH)**

**EP 0 257 503 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues Verfahren zur Herstellung von Hydrochinonderivaten, welche als Zwischenprodukte für die Herstellung von d-α-Tocopherol (natürliches Vitamin E) geeignet sind, sowie ein Verfahren zur Herstellung von d-α-Tocopherol selbst.

Es sind bereits einige Verfahren zur Herstellung von natürlichem Vitamin E bekannt, welche jedoch technisch nur von begrenztem Interesse sind, so dass natürliches Vitamin E bis heute nahezu ausschliesslich aus natürlichen Quellen extrahiert wird.

So verläuft eine Vielstufensynthese für Tocopherol ausgehend aus der Verbindung

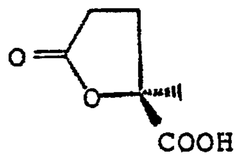

bzw.

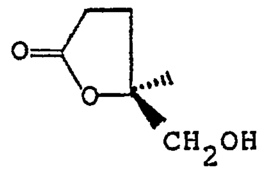

ebenfalls bereits über ein Hydrochinonderivat III (siehe US-PS 4 189 612).

Die grosse Anzahl Stufen dieser Synthese ergibt sich daraus, dass sowohl die erforderliche R-Konfiguration des C-2-Atom des Chromanrings über eine mehrstufige Reaktionsfolge eingebracht wird, wie auch der aromatische Ring des Chromansystems in mehrstufiger Sequenz aufgebaut wird.

Auch die Vielstufensynthese der Chromanringvorläufer der Formel

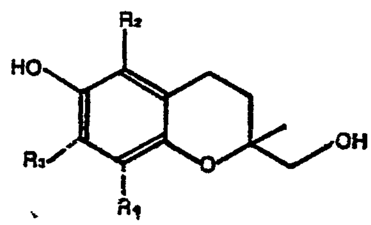

(7)

ausgehend aus Grignardreagenzien der Formel

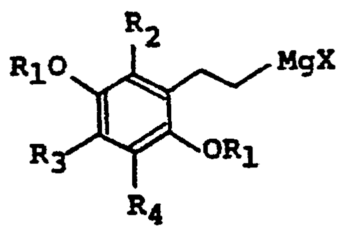

(2)

und den Steuerreagentien

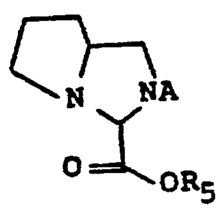

(1)

über

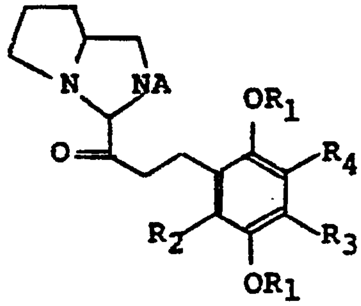

(3)

macht in der Folge von Zwischenprodukten der Formel III Gebrauch (EP-A1 065 368).

Die beträchtliche Anzahl von Stufen der Synthese ergibt sich primär daraus, dass die Komponente (1) welche die chirale Steuerfunktion trägt, in mehreren Stufen aus Prolin angebaut wird, und die Kopplung von (1) und (2) zur Ketoverbindung (3) die kontrollierte Einführung der erforderlichen R-Konfiguration am späteren $C_2$-Atom via diastereo selektive Alkylierung mittels Methylmagnesiumhalogenid erlaubt. Die Alkylierung bei äusserst tiefen Temperaturen (−100°C) zeitigt hohe Diastereoselektivität.

Optisch aktive Hydrochinonderivate III werden nach einer Methode neueren Datums direkt aus Verbindungen der Formel

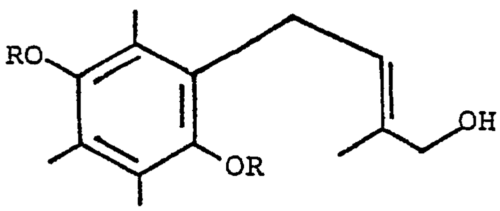

via Sharpless-oxydation erhalten (EP-A2 0 129 252).

Die weitere Umsetzung zu α-Tocopherol beinhaltet eine Kondensation mit einer $C_{15}$-Grignardverbindung und Cyclisation zu α-Tocopherol.

Ebenfalls nach einer neueren Methode werden die Hydrochinonderivate III gemäss EP-A1 0 173 142 durch Alkylierung von Ketoestern mittels Verbindungen der Formel

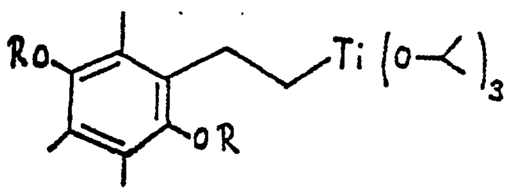

bei moderaten Temperaturen direkt erhalten. Die Ketoester tragen jeweils die Steuerkomponenten.

Es bestand somit ein Bedürfnis nach einem technisch realisierbaren Verfahren, gemäss welchem natürliches Vitamin E in guter Ausbeute und mit hoher optischer Reinheit erhalten werden kann. Dies ist nunmehr mittels des erfindungsgemässen Verfahrens möglich.

Dieses Verfahren ist dadurch gekennzeichnet, dass man eine Verbindung der allgemeinen Formel

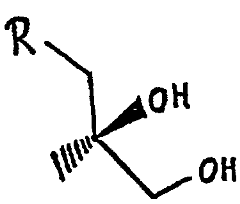

I

worin R eine Abgangsgruppe darstellt, mit einer Verbindung der allgemeinen Formel

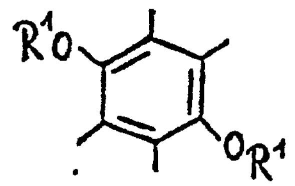

II

worin $R^1$ eine Aetherschutzgruppe darstellt, auf die in den Patentansprüchen angegebene Weise umsetzt und, gewünschtenfalls, eine so erhaltene Verbindung der allgemeinen Formel

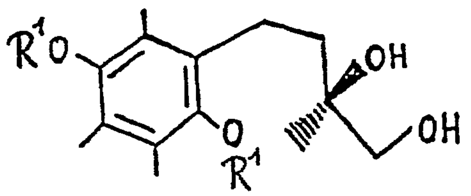

III

worin $R^1$ obige Bedeutung hat, in an sich bekannter Weise in d-α-Tocopherol überführt.

Der Ausdruck "Abgangsgruppe" bedeutet im Rahmen der vorliegenden Erfindung sowohl Halogen wie Chlor, Brom oder Jod, als auch Sulfonsäureester wie Tosylat oder Mesylat und dergleichen.

Der Ausdruck "Aetherschutzgruppe" bedeutet im Rahmen der vorliegenden Erfindung sowohl durch Hydrolyse spaltbare Gruppen wie etwa die Silylgruppen oder Alkoxymethylgruppen, beispielsweise die Methoxymethylgruppe, oder auch die Tetrahydropyranylgruppe, als auch oxidativ spaltbare Gruppen wie etwa $C_1$-$C_6$-Alkyl-äthergruppen. Weiterhin bedeutet das Zeichen

" 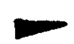 "

dass sich der entsprechende Rest oberhalb der Molekülebene befindet, während das Zeichen

" 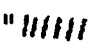 "

bedeutet, dass sich der entsprechende Rest unterhalb der Molekülebene befindet.

Die Umsetzung einer Verbindung der allgemeinen Formel I mit einer Verbindung der allgemeinen Formel II erfolgt erfindungsgemäss zweckmässig dergestalt, dass man entweder eine Verbindung der Formel I in einem inerten organischen Lösungsmittel mit einer Verbindung der Formel II in Gegenwart eines Alkali- oder Erdalkalimetallhydrids und eines Phasentransferkatalysators erhitzt oder zunächst eine Verbindung der Formel I in einem inerten organischen Lösungsmittel mit einem Alkalimetall- oder Erdalkalimetallhydrid bis zum Abklingen der Wasserstoffentwicklung reagieren lässt und anschliessend mit einer Verbindung der Formel II, nach Zugabe einer geeigneten Alkalialkyl- oder Erdalkalialkylverbindung zu letzterer in einem inerten organischen Lösungsmittel, umsetzt.

Als inerte organische Lösungsmittel können, im Rahmen der vorliegenden Erfindung, die üblicherweise bei metallorganischen Reaktionen verwendeten Lösungsmittel verwendet werden. Als Beispiele können hier genannt werden Aether, insbesondere cyclische Aether wie Tetrahydrofuran oder Dioxan oder auch Gemische dieser Aether mit aliphatischen Kohlenwasserstoffen wie insbesondere Pentan, Hexan und dergleichen. Als Alkali- oder Erdalkalihydride können im Rahmen der vorliegenden Erfindung insbesondere verwendet werden Lithium-, Natrium- oder Kaliumhydrid oder Calciumhydrid. Die im Rahmen der vorliegenden Erfindung benötigten Phasentransferkatalysatoren sind die üblicherweise verwendeten, bekannten Phasentransferkatalysatoren wie z.B. quaternäre Ammoniumsalze, Kronenäther oder auch Polyäther und dergleichen.

Als Alkalialkyl- oder Erdalkalialkylverbindungen kommen im Rahmen der vorliegenden Erfindung z.B. in Frage tert. Butyllithium, $C_1$-$C_6$-Alkylkalium, Grignard-Verbindungen und dergleichen.

Die Reaktion einer Verbindung der Formel I mit einer Verbindung der Formel II in Gegenwart eines Alkalimetall- oder Erdalkalimetallhydrides und eines Phasentransferkatalysators erfolgt unter Erhitzen auf eine Temperatur von etwa 60°C bis etwa Rückflusstemperatur des Reaktionsgemisches und gegebenenfalls unter Druck. Bei dieser Reaktion werden erfindungsgemäss wenigstens 3 Mol Hydrid pro Mol der Verbindungen der Formel I verwendet. Die Menge an Phasentransferkatalysator beträgt zweckmässig von etwa 1 bis etwa 10 Mol%. Die Reaktion einer Verbindung der Formel I mit einer Verbindung der Formel II in Abwesenheit eines Phasentransferkatalysators, d.h. die Reaktion einer Verbindung der Formel I mit einem Alkalimetall- oder Erdalkalimetallhydrid bis zum Abklingen der Wasserstoffentwicklung sowie auch die Reaktion einer Verbindung der Formel II mit einer geeigneten Alkalialkyl- oder Erdalkalialkylverbindung und auch die anschliessende Umsetzung können bei einer Temperatur von etwa –

20°C bis etwa +35°C, vorzugsweise bei etwa Raumtemperatur erfolgen. Auch tiefere Temperaturen sind hierbei möglich, jedoch aus ökonomischen Gründen nicht zweckmässig. Bei dieser Reaktion werden erfindungsgemäß wenigstens 2 Mol Hydrid pro Mol der Verbindungen der Formel I und 1 Mol Alkalialkyl- oder Erdalkalialkylverbindung pro Mol der Verbindungen der Formel II verwendet.

Die nach dem erfindungsgemässen Verfahren erhaltenen Verbindungen der allgemeinen Formel III sind bekannt und können in bekannter Weise in d-α-Tocopherol übergeführt werden, siehe z.B. Europ. Patentpublikation No. 129 252 vom 27.12.1984, Seiten 2 sea.

Die in dem erfindungsgemässen Verfahren als Ausgangsmaterial verwendeten Verbindungen der Formeln I und II sind bekannte Verbindungen oder Analoge bekannter Verbindungen, welche in zur Herstellung der bekannten Verbindungen analoger Weise hergestellt werden können.

Beispiel 1

2,4 g (19,6 mMol) (2R)-2-Methyl-3-chlorpropan-1,2-diol und 4,28 g (22m Mol) 2,5-Dimethoxy-1,3,4,6-tetramethylbenzol wurden in 20 ml Dioxan gelöst und mit 1,27 g (66 mMol) Natriumhydrid (80% in Mineralöl) und 0,1 ml Polyäthylenglycol 400 (als Phasentransferkatalysator) innert 1 Stunde auf Rückflusstemperatur erwärmt und 16 Stunden bei dieser Temperatur gerührt. Dann wurde das Gemisch mit 50 ml Wasser versetzt, filtriert und mit Wasser gewaschen. Der Rückstand wurde aus Aether/Hexan umkristallisiert und man erhielt 4,91 g (2S)-2-Methyl-4-(2,5-dimethoxy-3,4,6-trimethylphenyl)-1,2-butandiol in Form von weissen Kristallen mit einem Schmelzpunkt von 82-84°C und $[\alpha]_D^{20}$= +2,86° (c = 1,5% in $CHCl_3$).

Beispiel 2

Eine Lösung von 9.78 g (80 mMol) (2R)-2-Methyl-3-chlorpropan-1,2-diol in 100 ml Tetrahydrofuran wurde mit 4,3 g (180 mMol) Natriumhydrid bei Raumtemperatur durch portionenweise Zugabe unter Rühren während 1 Stunde umgesetzt. Zu der erhaltenen Suspension hat man bei Raumtemperatur eine Lösung von 88 mMol 2,5-Dimkethoxy-3,4,6-trimethylbenzyllithium (erhalten durch Umsetzung von 17,2 g (88 mMol) 2,5-Dimethoxy-1,3,4,6-tetramethylbenzol in 20 ml n-Pentan mit 88 mMol tert.Butyllithium bei Raumtemperatur) unter Rühren zugetropft und anschliessend noch 16 Stunden bei Raumtemperatur gerührt. Dann hat man unter Rühren mti 50 ml Wasser versetzt, im Vakuum (~15 mbar) die flüchtigen Anteile abdestilliert und das ausgefallene Material abfiltriert und mit Wasser gewaschen. Der Filterrückstand wurde aus Aether/Hexan umkristallisiert und man erhielt 23 g (2S)-2-Methyl-4-(2,5-dimethoxy-3,4,6-trimethylphenyl)-1,2-butandiol in Form von weissen Kristallen mit einem Schmelzpunkt von 83-85°C und $[\alpha]_D^{20}$= +2,86° (c = 1,5% in $CHCl_3$).

Das als Ausgangsmaterial verwendete (2R)-2-Methyl-3-chlorpropan-1,2-diol wurde wie folgt hergestellt:

7,9 ml (26 mMol) Tetraisopropyl-ortho-titanat, 150 mg (2,5 mMol) Calciumhydrid, 150 mg (2,5 mMol) Kieselgel und 5 ml (30 mMol) Dibutyl-D-tartrat wurden 10 Minuten bei -18°C in 150 ml Methylenchlorid stehen gelassen. Dann wurden 1,6 ml (25 mMol) beta-Methallylalkohol und 7 ml (50 mMol) Cumolhydroperoxid (66% in Cumol) zugetropft und das Gemisch 16 Stunden bei -18°C stehen gelassen. Hierauf wurden 300 ml Aethyläther und 50 ml (0,35 Mol) Natronlauge (28%ig) zugegeben und 1,5 Stunden bei Raumtemperatur gerührt. Dann wurde mit Aether extrabiert, die organischen Phasen mit 20,3 g (0.1 Mol) Magnesiumchlorid vesetzt und 16 Stunden bei Raumtemperatur gerührt. Anschliessend wurde filtriert, das Filtrat eingeengt und mit Wassedampf Cumol und Cumolalkohol abdestilliert. Der Rückstandwurde eingeengt und man erhielt 2,47 g (2R)-2-Methyl-3-chlorpropan-1,2-diol als farbloses Oel mit $[\alpha]_D^{20}$ = -5,4° (c = 3% in $CHCl_3$) und einer optischen Reinheit von über 98% (e.e.) nach gaschromatographischer Methode (Moscher-Derivat).

**Patentansprüche**

1. Verfahren zur Herstellung von Hydrochinonderivaten sowie von d-α-Tocopherol, dadurch gekennzeichnet, dass man eine Verbindung der allgemeinen Formel

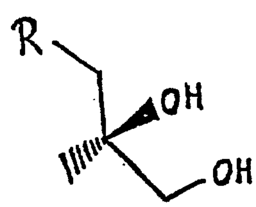

I

worin R eine Abgangsgruppe darstellt, mit einer Verbindung der allgemeinen Formel

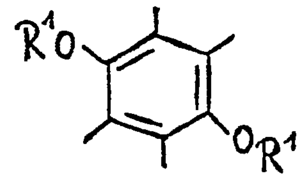

II

worin R¹ eine Aetherschutzgruppe darstellt, a) in einem inerten organischen Lösungsmittel in Gegenwart eines Alkalimetall- oder Erdalkalimetallhydrides und eines Phasentransferkatalysators unter Erhitzen und unter Verwendung von wenigstens 3 Mol Hydrid pro Mol der Verbindung I zu einer Verbindung der allgemeinen Formel

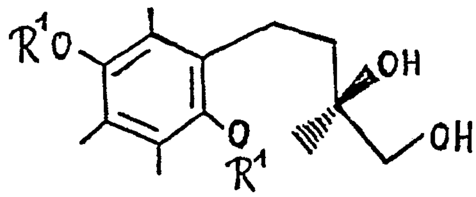

III

worin R¹ obige Bedeutung hat, umsetzt,
oder b) dass man die Verbindung der Formel I zunächst in einem inerten organischen Lösungsmittel mit einem Alkalimetall- oder Erdalkalimetallhydrid bis zum Abklingen der Wasserstoffentwicklung reagieren lässt und anschliessend mit einer Verbindung der Formel II, nach Zugabe einer geeigneten Alkalialkyl- oder Erdalkalialkylverbindung zu letzterer in einem inerten organischen Lösungsmittel unter Verwendung von wenigstens 2 Mol Hydrid pro Mol der Verbindung I und 1 Mol Alkalialkyl- oder Erdalkalialkylverbindung pro Mol der Verbindung der Formel II zu einer Verbindung der allgemeinen Formel III umsetzt,
und, gewünschtenfalls, die erhaltene Verbindung der allgemeinen Formel III in an sich bekannter Weise in d-α-Tocopherol überführt.

2. Verfahren nach Anspruch 1, dadurch gekennzeichnet, dass man die Umsetzung a) unter Erhitzen auf eine Temperatur von etwa 60°C bis etwa Rückflusstemperatur des Reaktionsgemisches durchführt.

3. Verfahren nach Anspruch 1, dadurch gekennzeichnet, dass man die Reaktion b) bei einer Temperatur von etwa –20°C bis etwa +35°C und vorzugsweise bei Raumtemperatur durchführt.

**Claims**

1. A process for the manufacture of hydroquinone derivatives and of d-α-tocopherol, characterized by reacting a compound of the general formula

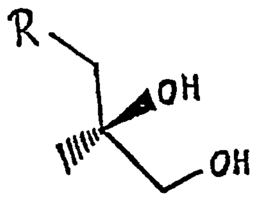

I

wherein R represents a leaving group, with a compound of the general formula

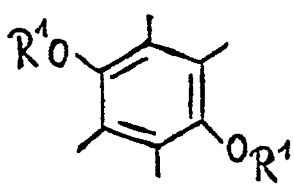

II

wherein R¹ represents an ether protecting group, a) in an inert organic solvent in the presence of an alkali metal hydride or alkaline earth metal hydride and a phase transfer catalyst while heating and using at least 3 mol of hydride per mol of compound I to give a compound of the general formula

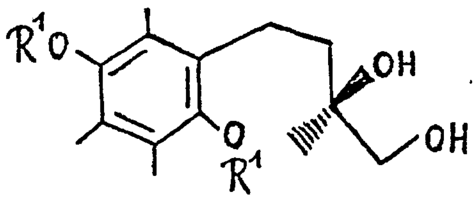

III

wherein $R^1$ has the above significance,
b) or firstly reacting the compound of formula I in an inert organic solvent with an alkali metal hydride or alkaline earth metal hydride until the hydrogen evolution has ended and subsequently reacting with a compound of formula II after the addition to the latter of a suitable alkali alkyl or alkaline earth alkyl compound in an inert organic solvent using at least 2 mol of hydride per mol of compound I and 1 mol of alkali alkyl or alkaline earth alkyl compound per mol of compound of formula II to give a compound of general formula III
and, if desired. converting the compound of general formula III obtained into d-α-tocopherol in a manner known per se.

2. A process according to claim 1, characterized in that the reaction a) is carried out while heating to a temperature from about 60°C to about the reflux temperature of the reaction mixture.

3. A process according to claim 1, characterized in that the reaction b) is carried out at a temperature from about −20°C to about +35°C and preferably at room temperature.

**Revendications**

1. Procédé de préparation de dérivés de l'hydroquinone et du d-α-tocophérol, caractérisé en ce que l'on fait réagir un composé de formule générale

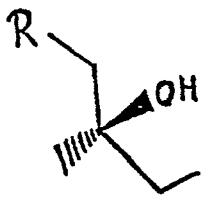

I

dans laquelle R représente un groupe éliminable, avec un composé de formule générale

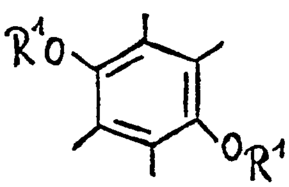

II

dans laquelle $R^1$ représente un groupe protecteur éther, a) dans un solvant organique inerte en présence d'un hydrure de métal alcalin ou alcalino-terreux et d'un catalyseur à transfert de phase, en chauffant, et en utilisant au moins trois moles d'hydrure par mole du composé I, ce qui donne un composé de formule générale

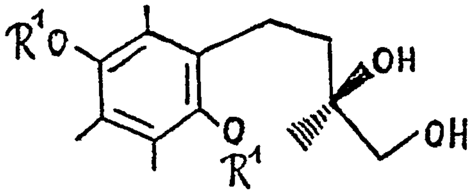

III

dans laquelle $R^1$ a les significations indiquées ci-dessus, ou bien
b) on fait d'abord réagir le composé de formule I dans un solvant organique inerte avec un hydrure de métal alcalin ou alcalino-terreux jusqu'à cessation du dégagement d'hydrogène, puis avec un composé de formule II, après addition d'un dérivé alkylé approprié de métal alcalin ou alcalino-terreux à ce dernier dans un solvant organique inerte, en utilisant au moins deux moles d'hydrure par mole du composé I et

une mole de dérivé alkylé de métal alcalin ou alcalino-terreux par mole du composé de formule II, ce qui donne un composé de formule générale III,
et, si on le désire, on convertit le composé de formule générale III ainsi obtenu, de manière connue en soi, en le d-α-tocophérol.

2. Procédé selon la revendication 1, caractérisé en ce que la réaction a) est effectuée en chauffant à une température allant d'environ 60°C jusqu'aux environs de la température de reflux du mélange de réaction.

3. Procédé selon la revendication 1, caractérisé en ce que la réaction b) est effectuée à une température allant d'environ –20°C jusqu'à environ +35°C, de préférance à température ambiante.